# FASCICULE DE BREVET EUROPEEN

(11) **EP 3 302 645 B1**
(45) Date de publication et mention de la délivrance du brevet: **31.03.2021**
(21) Numéro de dépôt: 16736902.4
(22) Date de dépôt: 02.06.2016
(51) Int. Cl.: A61M 5/315, A61M 5/20, A61M 5/32

(54) **AUTOINJECTEUR**
AUTOMATISCHER INJEKTOR
AUTO-INJECTOR

(30) Priorité: 05.06.2015 FR 1555163
(43) Date de publication de la demande: 11.04.2018
(73) Titulaire: Aptar France SAS, 27110 Le Neubourg (FR)
(72) Inventeur: FABIEN, David, 29810 Plouarzel (FR); GOMEZ, Thomas, 33160 Saint Aubin De Medoc (FR); HIS, Olivier, 27430 Saint Etienne Du Vauvray (FR); SAUSSAYE, Anthony, 61300 Saint Sulpice Sur Risle (FR)
(74) Mandataire: CAPRI
(86) Numéro de dépôt international: PCT/FR2016/051314
(87) Numéro de publication internationale: WO 2016/193624

(56) Documents cités:
- WO-A1-2011/101380
- WO-A1-2013/178512
- WO-A2-2015/073740
- US-A1- 2013 218 093

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois un certain nombre d'inconvénients.

Ainsi, notamment lorsque le volume de produit fluide est relativement important et/ou lorsque le produit fluide injecté est relativement visqueux, il est souhaitable de permettre au produit de diffuser du site d'injection pendant quelques secondes après ladite injection. Si l'utilisateur retire l'autoinjecteur immédiatement après la fin de l'injection, une partie du produit peut ressortir du corps de l'utilisateur ce qui diminue l'efficacité du traitement. Il est donc souhaitable de prévoir que l'utilisateur maintienne l'autoinjecteur contre son corps encore pendant quelques secondes après la fin de l'injection. Cet aspect est généralement résolu par les autoinjecteurs existants par la notice d'utilisation qui demande à l'utilisateur de compter dans sa tête un certain nombre de secondes avant de retirer le dispositif. Ceci n'est pas fiable et donc insatisfaisant, car le système dépend alors de l'utilisateur lui-même, qui dans certains cas peut être perturbé ou affaibli par l'action d'injection qu'il vient de réaliser.

Les documents WO2013178512, WO2015073740, WO2011101380 et US2013218093 décrivent des autoinjecteurs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable d'utilisation, qui permet à l'utilisateur de déterminer quand il doit retirer ou quand il peut retirer l'autoinjecteur de son corps après son utilisation, qui soit sûr et qui empêche tout risque de blessure, et qui soit simple et peu coûteux à fabriquer et à assembler.

La présente invention a donc pour objet un autoinjecteur tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1a est une vue schématique de côté d'un autoinjecteur selon un mode de réalisation avantageux, en position de repos avant piquage
Les figures 1b et 1c sont des vues similaires à celle de la figure 1a, en section transversale, selon deux plans de coupe différents,
Les figures 2a à 2c sont des vues similaires à celles des figures 1a à 1c, en position après piquage et avant injection,
Les figures 3a à 3c sont des vues similaires à celles des figures 2a à 2c, en position juste avant la fin d'injection et au moment du déclenchement du système retardateur,
Les figures 4a et 4b sont des vues similaires à celles des figures 3a et 3c, en début d'actionnement du système retardateur,
Les figures 5a à 5c sont des vues similaires à celles des figures 3a à 3c, en fin d'actionnement du système retardateur,
Les figures 6a à 6c sont des vues similaires à celles des figures 3a à 3c, en fin d'actionnement du dispositif d'indication et avant retrait de l'autoinjecteur du site d'injection,
La figure 7 est une vue similaire à celle de la figure 6a, en position de fin d'utilisation, après retrait de l'autoinjecteur du site d'injection,
La figure 8 est une vue en perspective éclatée du système retardateur selon ledit mode de réalisation des figures 1 à 7,
Les figures 9 et 10 sont des vues partielles en perspective découpée du système de la figure 8, montrant l'unité formée par le réservoir de fluide et le piston, respectivement avant et en cours d'actionnement du système retardateur,
Les figures 11 et 12 sont des vues de détail en perspective de l'unité formée par le réservoir de fluide et le piston, après actionnement du système retardateur, respectivement avant et après déformation des pattes déformables du réservoir,
Les figures 13 et 14 sont des vues de détail similaires à celles des figures 11 et 12, mais en section transversale, respectivement avant et après déformation des pattes flexibles du réservoir,
La figure 15 est une vue schématique en section horizontale, selon le plan de coupe A-A de la figure 14,
Les figures 16 à 18 sont des vues de détail en perspective découpée du système de la figure 8, respectivement avant actionnement, après actionnement et en fin d'actionnement du système retardateur,
La figure 19 est une vue similaire à celles des figures 16 à 18, en fin d'actionnement du dispositif d'indication,
Les figures 20a et 20b sont des vues schématiques, respectivement de côté et en section transversale, d'un autoinjecteur selon un mode de réalisation avantageux de la présente invention, en position de repos avant piquage,
Les figures 21a et 21b sont des vues similaires à celles des figures 20a et 20b, en position après piquage et avant injection,
Les figures 22a et 22b sont des vues similaires à celles des figures 21a et 21b, en position juste avant la fin d'injection et au moment du déclenchement du système retardateur,
Les figures 23a et 23b sont des vues similaires à celles des figures 22a et 22b, en début d'actionnement du système retardateur,
Les figures 24a et 24b sont des vues similaires à celles des figures 23a et 23b, en fin d'actionnement du système retardateur,
La figure 24c est une vue similaire à celle de la figure 24b selon un autre plan de coupe,
Les figures 25a à 25c sont des vues similaires à celles des figures 24a à 24c, en fin d'actionnement du dispositif d'indication et avant retrait de l'autoinjecteur du site d'injection,
La figure 26 est une vue similaire à celle de la figure 25a, en position de fin d'utilisation, après retrait de l'autoinjecteur du site d'injection,
La figure 27 est une vue en perspective éclatée du système retardateur selon ledit mode de réalisation des figures 20 à 26,
Les figures 28 à 30 sont des vues partielles en perspective découpée du système de la figure 27, montrant le sous-ensemble formé du réservoir, du piston, de la clé de verrouillage et du ressort, respectivement en début, en cours et en fin d'actionnement du système retardateur,
Les figures 31 à 33 sont des vues de détail en perspective découpée du système de la figure 27, respectivement avant actionnement, après actionnement et en fin d'actionnement du système retardateur,
La figure 34 est une vue similaire à celles des figures 31 à 33, en fin d'actionnement du dispositif d'indication.

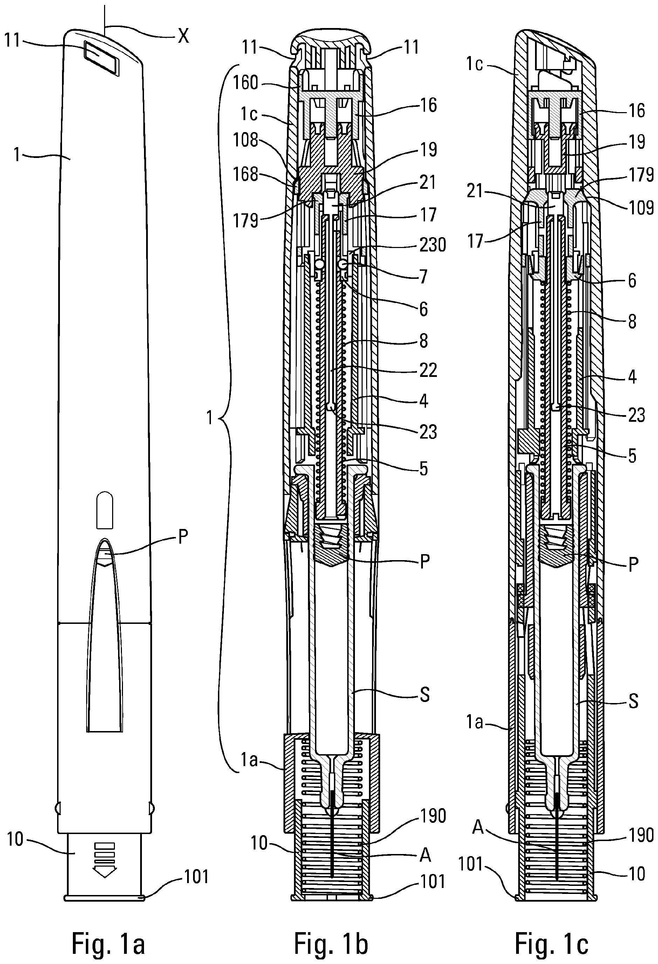

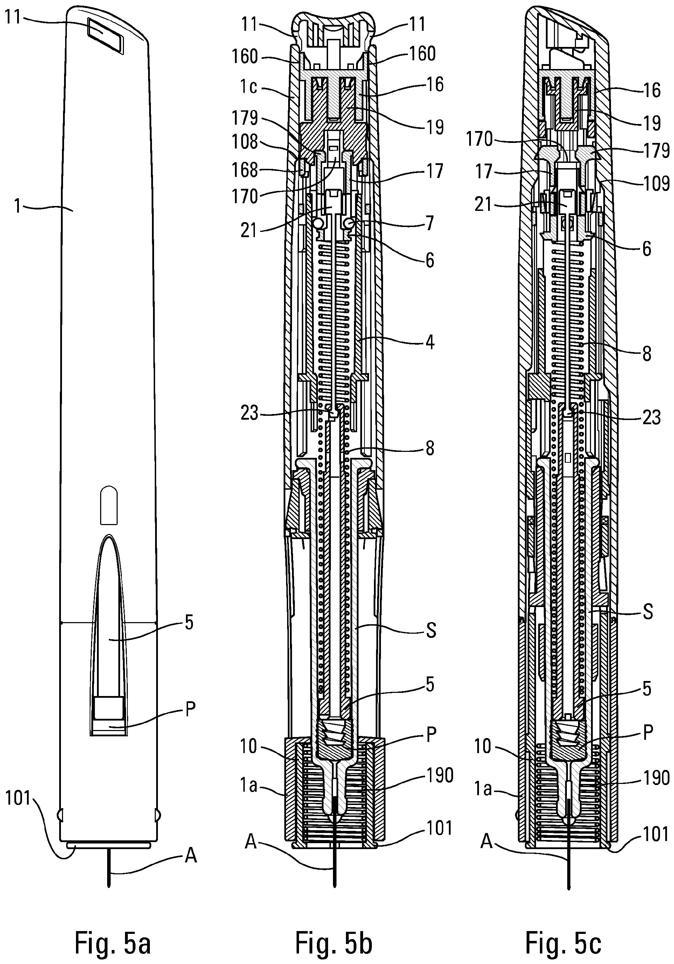

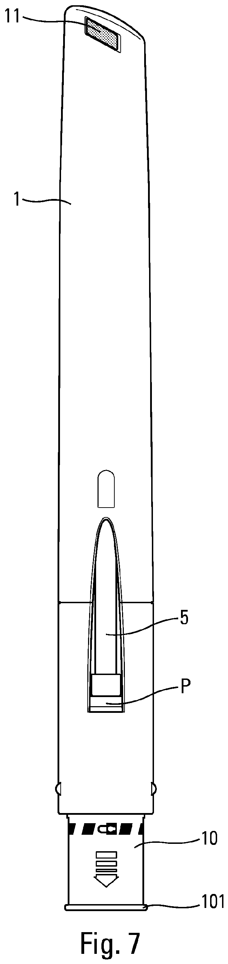

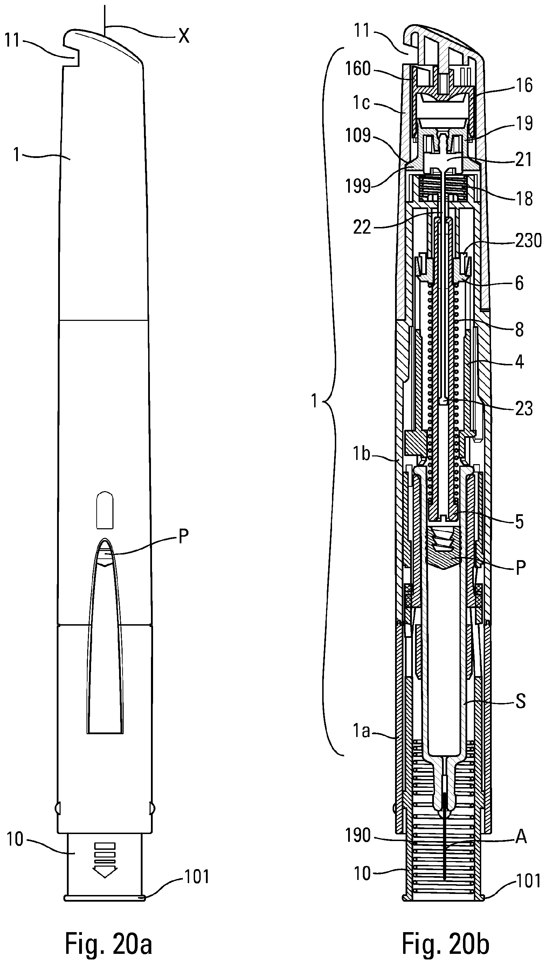

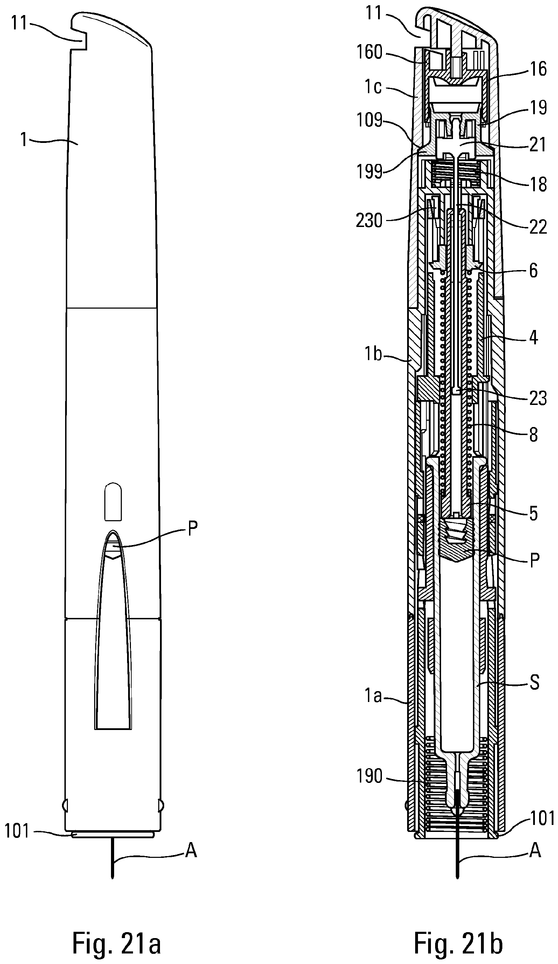

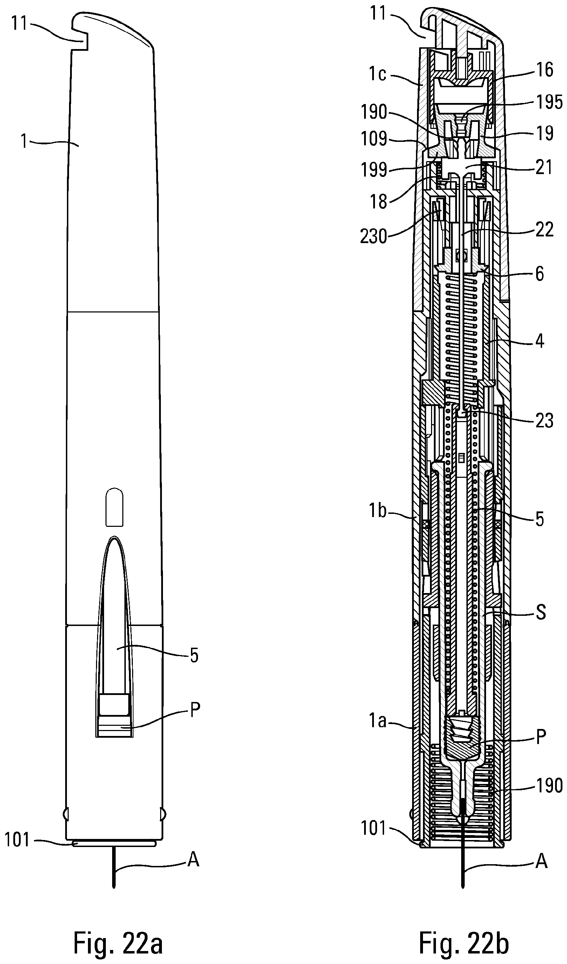

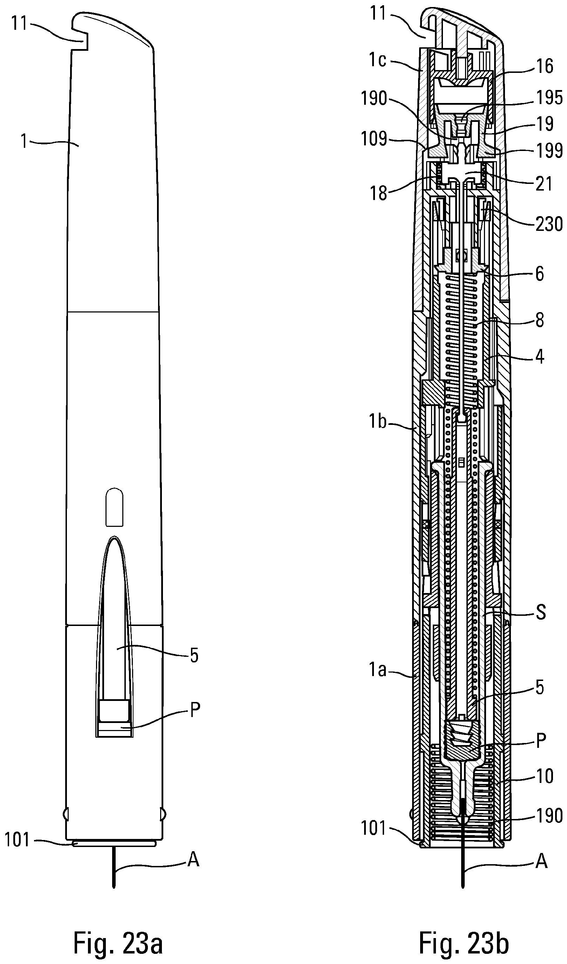

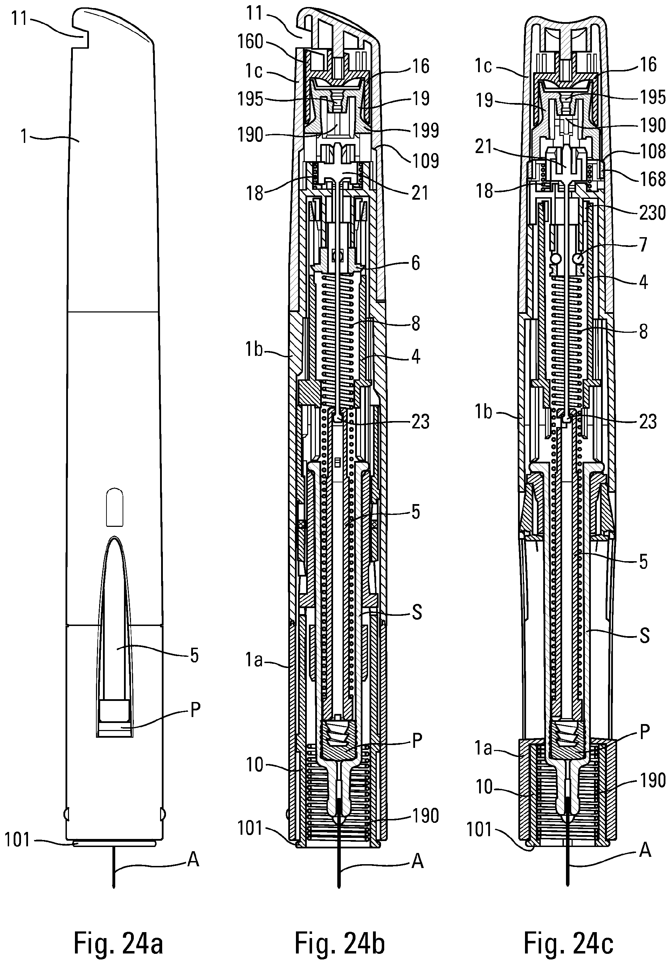

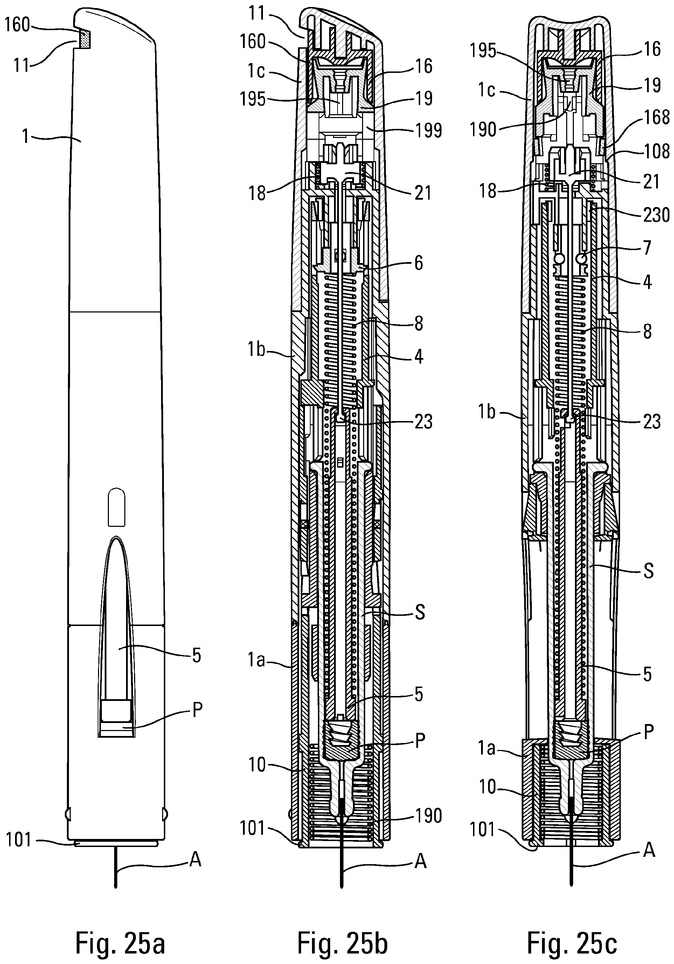

Dans la description ci-après, les termes "supérieur", "inférieur", "haut" et "bas" se réfèrent aux positions représentées sur les figures 1a à 7, 17, 18 et 20a à 26. Les termes "axial" et "radial" se réfèrent à l'axe central longitudinal X, représenté notamment sur les figures 1a et 20a, qui correspond à l'axe longitudinal de l'aiguille.

L'autoinjecteur va être décrit ci-après en référence à deux modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

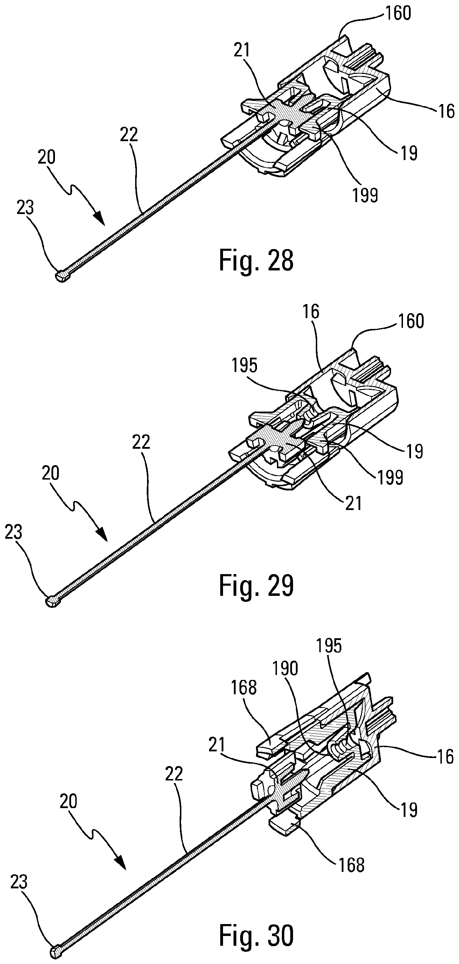

L'autoinjecteur représenté sur les figures comporte un corps 1 dans lequel coulisse axialement un manchon actionneur 10, dont l'extrémité inférieure 101 est destinée à venir en contact avec le corps du patient autour de la zone d'injection. Dans l'exemple des figures 1 à 19, l'autoinjecteur comporte un corps inférieur 1a et un corps supérieur 1c qui sont assemblés pour former le corps 1 de l'autoinjecteur. Dans l'exemple des figures 20 à 34, l'autoinjecteur comporte un corps inférieur 1a, un corps intermédiaire 1b et un corps supérieur 1c qui sont assemblés pour former le corps 1 de l'autoinjecteur. Ci-après, le terme "corps" et la référence numérique "1" seront utilisés pour désigner ledit corps unitaire formé par l'assemblage dudit corps inférieur 1a avec ledit corps intermédiaire 1b et/ou ledit corps supérieur 1c. Il est à noter que le corps 1 pourrait être formé d'un nombre quelconques de parties de corps, et que les modes de réalisation des figures, avec deux ou trois parties de corps, ne sont pas limitatifs.

Un réservoir S peut être inséré dans ledit corps 1 de l'autoinjecteur, ledit réservoir S étant de préférence fixe dans ledit corps 1. Ce réservoir S contient du produit fluide, et comporte un piston P et une aiguille A. Le piston P est adapté à se déplacer dans ledit réservoir S pour injecter le produit fluide à travers ladite aiguille A. Eventuellement, la présente invention pourrait aussi s'appliquer à un réservoir sans aiguille, notamment dans un dispositif d'injection sans aiguille.

La présente description sera faite en référence à une seringue S, qui peut être de tout type. Plus généralement, il est entendu que le terme "seringue" dans la présente description englobe tout type de réservoir associé à une aiguille. De préférence, le réservoir S est une seringue pré-remplie.

Avant actionnement de l'autoinjecteur, l'aiguille A de la seringue S peut être protégée par un capuchon (non représenté), l'autoinjecteur pouvant comporter un capot (non représenté) que l'utilisateur peut retirer avant actionnement. Le retrait du capot provoque avantageusement le retrait du capuchon.

Avant actionnement, le manchon actionneur 10 est dans une première position projetée dans laquelle il entoure l'aiguille A, comme représenté sur les figures 1a et 1b d'une part et 20a et 20b d'autre part. Lors de l'actionnement, le manchon actionneur 10 coulisse à l'intérieur du corps 1 vers une position d'actionnement, pour exposer l'aiguille A et permettre le piquage puis l'injection du produit fluide.

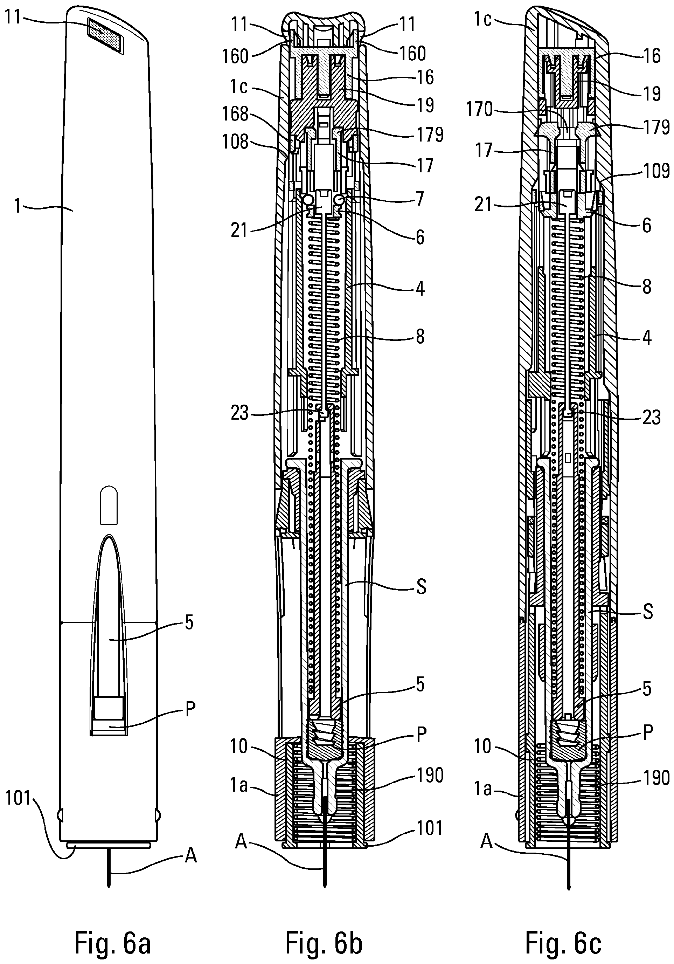

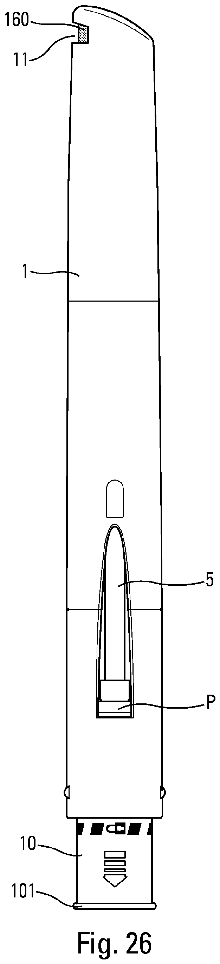

Après l'injection, lorsque l'utilisateur retire l'autoinjecteur du site d'injection, le manchon actionneur 10 revient dans une seconde position projetée de fin d'utilisation, dans laquelle il est à nouveau disposé autour de l'aiguille A, pour éviter tout risque de blessure avec ladite aiguille, comme représenté sur les figures 6 et 26.

Le manchon actionneur 10 est avantageusement sollicité vers ses positions projetées par un organe élastique ou ressort 190, qui peut être de type quelconque. Avantageusement, dans ladite position de fin d'utilisation, ledit manchon actionneur 10 est verrouillé, et ne peut plus être déplacé axialement vers l'intérieur dudit corps 1. Ce verrouillage peut par exemple être réalisé par des pattes (non représentées), solidaires du corps 1 ou du réservoir S, et coopérant avec des ouvertures (non représentées) dudit manchon actionneur 10 lorsque celui-ci atteint sa seconde position projetée. Ce verrouillage, qui n'est pas essentiel au fonctionnement de la présente invention, ne sera pas décrit plus en détail ci-après. Il pourrait être réalisé différemment du mode de réalisation particulier mentionné ci-dessus. En particulier, il pourrait être réalisé conformément aux enseignements des documents WO2013175140 ou WO2013175142.

L'autoinjecteur comporte également un système d'injection automatique, comportant notamment une tige de piston 5 adaptée à coopérer avec le piston P pour le déplacer dans le réservoir S pour distribuer le produit fluide à travers l'aiguille A. Cette tige de piston 5 est classiquement sollicitée par un ressort d'injection 8 vers sa position de distribution, et retenue avant actionnement dans sa position de repos par une serrure d'injection appropriée.

Une serrure d'injection avantageuse est notamment décrite dans le document WO2015155484.

La serrure peut comprendre au moins un élément de blocage 7 retenu en position de blocage par une bague de blocage 230 fixée, notamment encliquetée sur un organe support 6 sur lequel s'appuie le ressort d'injection 8. Le déclenchement de ladite serrure d'injection provoque l'actionnement desdits moyens d'injection et donc l'injection du produit fluide à travers l'aiguille. Ladite serrure d'injection peut en outre comporter un manchon de commande 4 disposé dans ledit corps 1, ledit manchon de commande 4 contenant ladite tige de piston 5 et ledit ressort d'injection 8, ladite tige de piston 5 comportant un évidement radial recevant au moins un élément de blocage 7 mobile entre une position de blocage et une position de déblocage. Ledit au moins un élément de blocage 7 est de préférence de forme sensiblement sphérique, tel qu'une bille. Avantageusement, lesdites billes sont sollicitées radialement vers l'extérieur par ladite tige de piston 5 et sont retenues en position de blocage par la bague de blocage 230. Cette bague de blocage 230 est déplaçable axialement par rapport à ladite tige de piston 5 et par rapport audit organe support 6 entre une position de verrouillage, dans laquelle elle maintient lesdites billes en position de blocage, et une position de déverrouillage, dans laquelle lesdites billes sont libérés pour ainsi débloquer ladite serrure d'injection, permettant audit ressort d'injection 8 de déplacer ladite tige de piston 5 vers sa position d'injection. La bague de blocage 230 peut notamment être déplacée vers sa position de déverrouillage par ledit manchon de commande 4.

Lorsque l'aiguille A de la seringue S a pénétré le corps de l'utilisateur, la bague de blocage 230 est déplacée axialement vers le haut, ce qui a pour effet de libérer les billes 7 de leur position de blocage, celles-ci étant alors déplacées radialement vers l'extérieur. La tige de piston 5 n'est alors plus retenue par les billes et elle est donc déplacée axialement vers le bas pour réaliser l'injection du produit.

L'autoinjecteur comporte un dispositif d'indication visuel, sonore et/ou tactile pour indiquer à l'utilisateur, notamment par un son audible, par une vibration et/ou par une indication visuelle et/ou tactile, qu'il peut retirer l'autoinjecteur du site d'injection. Ledit dispositif d'indication visuel, sonore et/ou tactile est de préférence disposé à l'extrémité arrière dudit corps 1, opposée audit site d'injection. En particulier, dans les exemples représentés, le dispositif d'indication comporte un élément indicateur qui donne une indication visuelle, par un affichage 160 adéquat dans une ou plusieurs fenêtre(s) 11 du corps 1. Avantageusement une indication sonore et/ou tactile peut également être fournie, comme cela sera décrit plus en détails ci-après.

Pour éviter que l'utilisateur ne retire l'autoinjecteur du site d'injection dès la fin d'injection, l'autoinjecteur comporte un système retardateur, qui va retarder l'actionnement dudit dispositif d'indication par rapport à la fin de l'injection.

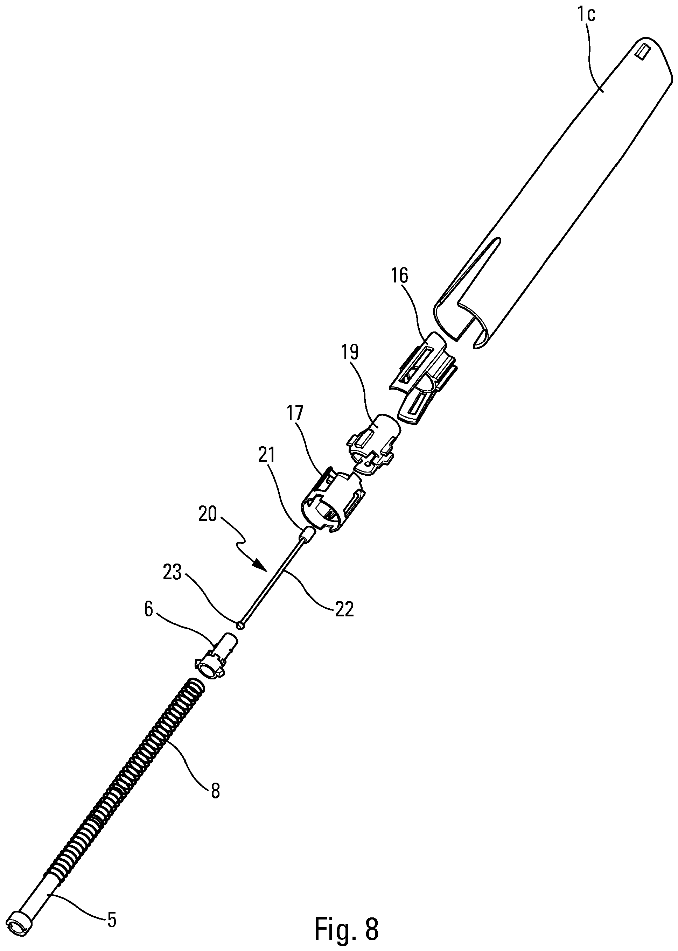

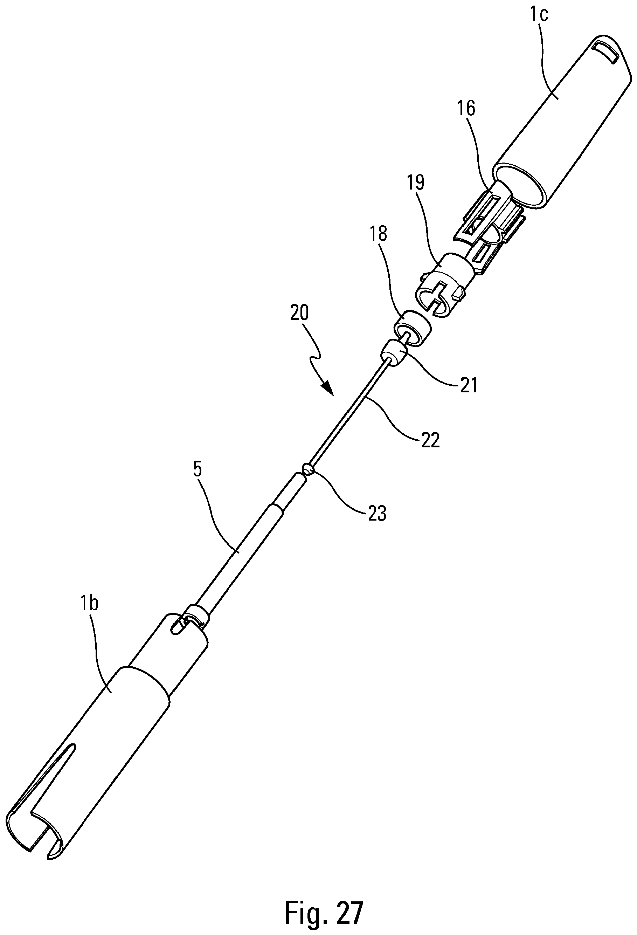

Les figures 8 à 19 illustrent un système retardateur selon un mode de réalisation avantageux, et les figures 27 à 34 illustrent un système retardateur selon un mode de réalisation avantageux de l'invention.

Ce système retardateur a principalement pour but de décaler dans le temps l'indication visuelle, sonore et/ou tactile après la fin de l'injection du produit fluide à l'intérieur dudit corps. Ceci permet notamment une diffusion pendant quelques secondes du produit après son injection. Un tel retardateur procure aussi un bénéfice pour l'utilisateur, qui n'a plus à compter, par exemple jusqu'à 10, après son injection, le temps pris pour ce comptage pouvant être très variable d'un utilisateur à un autre. Avec un retardateur, on facilite la séquence d'utilisation d'un autoinjecteur.

Le retardateur mécanique permet de décaler ainsi de quelques secondes cette indication de fin d'utilisation par rapport à la fin de l'injection, ce retard étant prédéterminable.

L'invention exploite le phénomène de transfert de fluide pour générer ledit retard, et utilise un réservoir 16, un piston 19 coulissant axialement dans ledit réservoir 16, et un fluide disposé dans ledit réservoir 16, et adapté à être déplacé par ledit piston 19, lorsqu'il se déplace dans ledit réservoir 16.

Dans les exemples représentés, pendant l'actionnement du système retardateur, le réservoir 16 est fixe par rapport au corps 1, et le piston 19 est axialement déplaçable par rapport audit réservoir 16. L'inverse est toutefois aussi envisageable.

Un fluide est disposé dans ledit réservoir 16, axialement au-dessus dudit piston 19, ledit fluide étant adapté à s'écouler hors dudit réservoir lors du déplacement dudit piston 19 dans ledit réservoir 16. Le fluide peut s'écouler dans une autre chambre du réservoir ou simplement hors dudit réservoir. Il peut s'écouler à travers un ou plusieurs passages d'écoulement, dimensionné(s) pour générer un freinage du déplacement dudit piston 19 dans ledit réservoir 16.

Selon la viscosité du fluide contenu dans le réservoir 16 et/ou selon les formes et dimensions du réservoir 16, du piston 19 et/ou du ou des passage(s) d'écoulement, on peut régler assez précisément ledit freinage et donc le temps entre le début et la fin d'actionnement du système retardateur. En fin d'actionnement du système retardateur, le réservoir 16 peut se déplacer axialement dans le corps 1 pour réaliser l'indication, et notamment indiquer dans la fenêtre 11 de l'indicateur que l'autoinjecteur peut être retiré du site d'injection. L'actionnement du dispositif d'indication visuel, sonore et/ou tactile est donc retardée par rapport à la fin de l'injection, ce qui permet au produit injecté de se diffuser dans le site d'injection pendant cette durée de retard.

La figure 8 illustre une vue schématique en perspective éclatée de ce système retardateur selon un mode de réalisation avantageux. Celui-ci comprend le corps supérieur 1c, le réservoir 16 contenant un fluide approprié, le piston 19 coulissant dans ledit réservoir 16, un élément pousseur 17, une clé de verrouillage 20, la tige de piston 5, l'organe support 6 et le ressort d'injection 8. L'élément pousseur 17 est avantageusement fixé, notamment encliqueté sur ledit organe support 6 de la serrure d'injection.

Dans ce mode de réalisation, le corps 1 n'est composé que de deux parties, une partie de corps inférieur 1a et une partie de corps supérieur 1c. Il n'y a donc pas dans ce mode de réalisation de partie de corps intermédiaire.

Dans l'exemple représenté, le réservoir 16 forme également l'élément indicateur du dispositif d'indication visuel, sonore et/ou tactile. Avantageusement, ledit réservoir 16 peut comporter une projection 160 approprié pour venir indiquer la fin d'utilisation de l'autoinjecteur dans une ou plusieurs fenêtres 11 du corps 1, notamment du corps supérieur 1c. Dans l'exemple représenté sur les figures 1 à 19, le corps 1 comporte une seule fenêtre 11.

L'élément pousseur 17 est déplaçable axialement dans ledit corps 1, et coopère avec ledit piston 19. Pendant le déplacement axial dudit piston 19, le réservoir 16 est bloqué contre tout déplacement axial. Ainsi, un déplacement axial dudit élément pousseur 17 va provoquer un déplacement axial du piston 19 dans le réservoir 16. En variante, on pourrait envisager l'inverse, à savoir que l'élément pousseur 17 déplace un réservoir mobile par rapport à un piston fixe.

La clé de verrouillage 20 comporte une tête 21 adaptée à coopérer avec le système retardateur, une tige longitudinale 22, et en embout 23 adapté à coopérer avec la tige de piston 5.

En position avant déclenchement du système retardateur, la tête 21 de la clé de verrouillage 20 est en position de blocage dans laquelle elle coopère avec un évidement axial 170 de l'élément pousseur 17, de sorte que celui-ci est bloqué en translation par ladite clé. Lorsque la tige de piston 5 arrive vers sa position de fin d'injection, elle va coopérer avec l'embout 23 de la clé de verrouillage 20, et ainsi tirer ladite clé axialement vers le bas. De ce fait, la tête 21 de ladite clé de verrouillage 20 sort axialement dudit évidement 170, de sorte que ledit élément pousseur 17 n'est plus bloqué en translation par ladite clé 20.

Le ressort d'injection 8 sollicite l'élément pousseur 17 axialement en translation en direction de l'arrière dudit corps supérieur 1c. Tant que l'élément pousseur 17 est bloqué par ladite clé de verrouillage 20, le système retardateur est donc également bloqué.

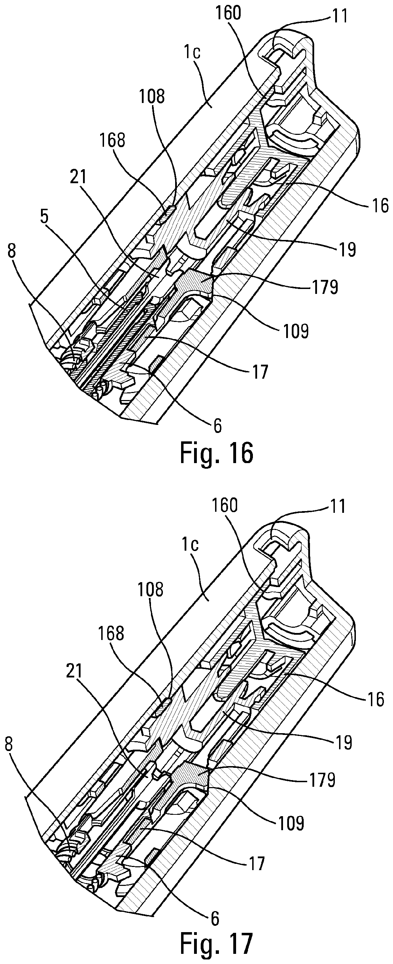

Dans l'exemple représenté, avant actionnement du système retardateur, l'élément pousseur 17 est bloqué contre tout déplacement axial vers le haut par une première paroi tronconique ou inclinée 109 du corps supérieur 1c qui coopère avec des pattes flexibles 179 dudit élément pousseur 17. Dans l'exemple représenté, il y a deux pattes flexibles 179 diamétralement opposées, mais on pourrait envisager un nombre différent de premières pattes flexibles, par exemple une seule ou plus de deux pattes. Comme visible notamment sur la figure 16, en position de blocage, ladite tête 21 de la clé de verrouillage 20 empêche la déformation radiale vers l'intérieur desdites pattes flexibles 179 de l'élément pousseur 17. Lorsque ladite tête 21 n'est plus en position de blocage, lesdites pattes flexibles 179 peuvent se déformer radialement vers l'intérieur, sous l'effet du ressort d'injection 8 qui pousse ledit élément pousseur 17 axialement vers le haut, et du fait de la coopération desdites pattes flexibles 179 avec la première paroi inclinée 109 du corps 1.

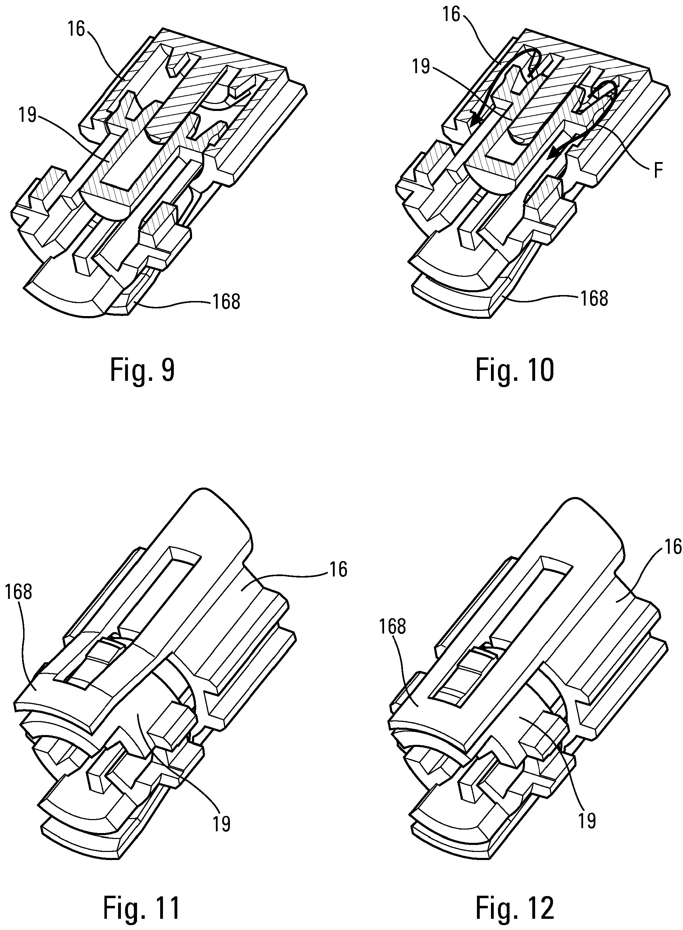

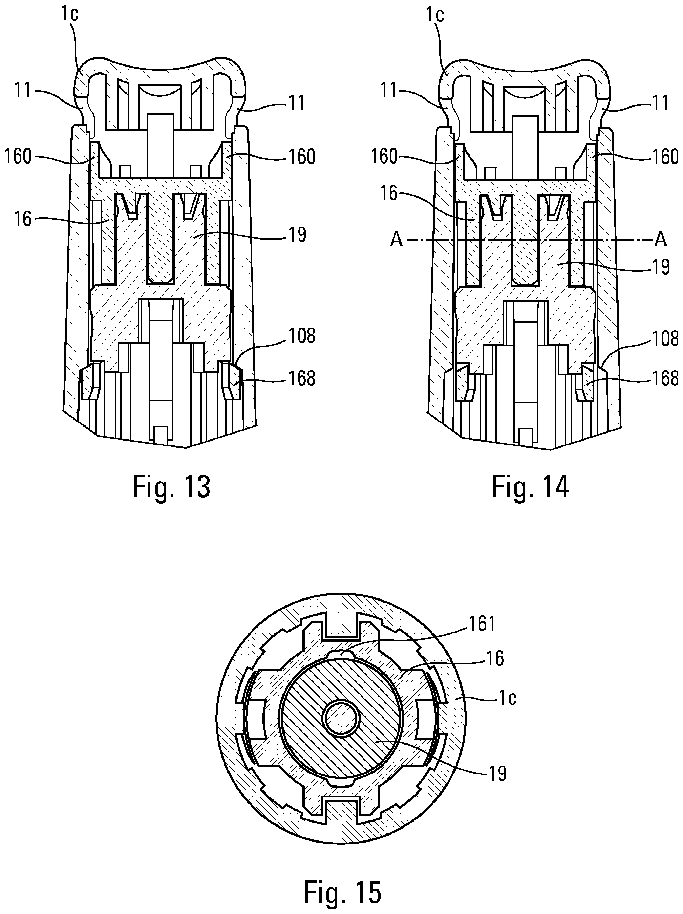

Lorsque ladite clé de verrouillage 20 libère ledit élément pousseur 17 en fin d'injection, celui-ci est donc déplacé axialement par ledit ressort d'injection 8. Le système retardateur est alors déclenché, avec ledit l'élément pousseur 17 coopérant avec ledit piston 19 pour le déplacer axialement dans ledit réservoir 16. Ceci provoque le transfert du fluide hors dudit réservoir 16 à travers des passages d'écoulement 161 appropriés. Dans l'exemple représenté, ces passages d'écoulement sont formés par des gorges axiales formées dans ledit réservoir 16, notamment visibles sur la figure 15. Les flèches F sur la figure 10 illustrent ce transfert de fluide. La vitesse de déplacement axial de l'élément pousseur 17 correspond donc à la vitesse de déplacement axial dudit piston 19, qui subit un freinage dû au transfert du fluide hors du réservoir 16 à travers lesdits passages d'écoulement 161.

Pendant le déplacement dudit piston 19 dans ledit réservoir 16, ce dernier est bloqué contre tout déplacement axial par des pattes flexibles 168 dudit réservoir 16 qui coopèrent avec une seconde paroi inclinée 108 du corps 1, comme visible sur la figure 13. Dans l'exemple représenté, il y a deux pattes flexibles 168 diamétralement opposées, mais on pourrait envisager un nombre différent de pattes flexibles, par exemple une seule ou plus de deux pattes. Comme visible notamment sur les figures 16 et 17, tant que le piston 19 n'a pas terminé son déplacement axial dans ledit réservoir 16, il empêche la déformation radiale vers l'intérieur desdites pattes flexibles 168 du réservoir 16. Lorsque ledit piston 19 a terminé son déplacement axial dans ledit réservoir 16, il ne bloque plus lesdites pattes flexibles 168, qui peuvent alors se déformer radialement vers l'intérieur, sous l'effet du ressort d'injection 8 qui pousse l'unité formée par ledit élément pousseur 17, ledit piston 19 et ledit réservoir 16 axialement vers le haut, et du fait de la coopération desdites pattes flexibles 168 avec la seconde paroi inclinée 108 du corps 1. Ceci est notamment visible sur la figure 14.

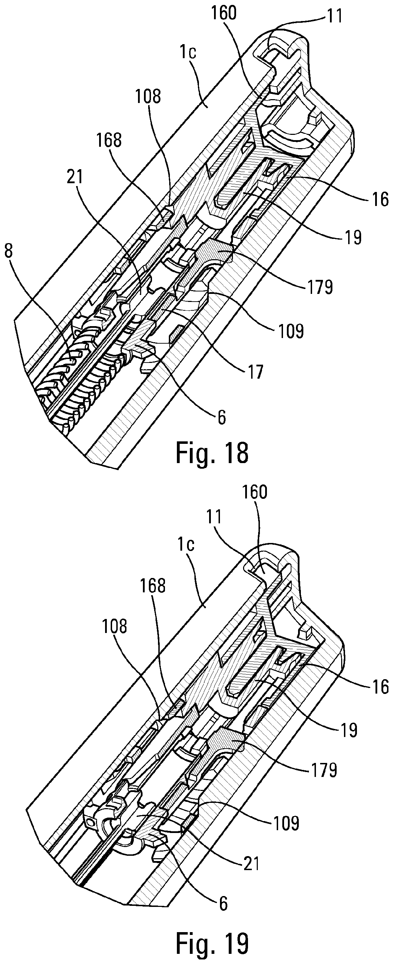

Lorsque ledit piston 19 a terminé son déplacement axial dans ledit réservoir 16, celui-ci est donc libéré de son blocage, et l'unité formée de l'élément pousseur 17, du piston 19 et du réservoir 16 est alors projetée par le ressort d'injection 8 axialement contre le fond du corps supérieur 1c, comme illustré sur la figure 19. La partie d'indication 160 du réservoir 16 est alors disposée face à la fenêtre d'indication 11 du corps 1. Ce déplacement non freiné ou projection de ladite unité permet aussi de générer une indication sonore, par contact entre le réservoir 16, le piston 19 et/ou l'élément pousseur 17 avec le corps 1. Cette indication sonore peut être réalisée au niveau de ladite partie d'indication 160 ou sur une autre partie de l'unité formée par l'élément pousseur 17, le piston 19 et le réservoir 16, adaptée à coopérer avec une partie appropriée du corps 1. Il est à noter que le choc générant l'indication sonore peut aussi fournir une indication tactile en générant une vibration de l'autoinjecteur dans la main de l'utilisateur, utile notamment pour les malentendants.

La figure 27 illustre une vue schématique en perspective éclatée de ce système retardateur selon un mode de réalisation avantageux de l'invention. Celui-ci comprend le corps supérieur 1c, le réservoir 16 contenant un fluide approprié, le piston 19 disposé dans ledit réservoir 16, un ressort retardateur 18, une clé de verrouillage 20, la tige de piston 5 et le corps intermédiaire 1b.

Dans l'exemple représenté, le réservoir 16 forme également l'élément indicateur du dispositif d'indication visuel, sonore et/ou tactile. Avantageusement, ledit réservoir 16 peut comporter une projection 160 approprié pour venir indiquer la fin d'utilisation de l'autoinjecteur dans une ou plusieurs fenêtres 11 du corps 1, notamment du corps supérieur 1c. Dans l'exemple représenté sur les figures 20 à 34, le corps 1 comporte une seule fenêtre 11.

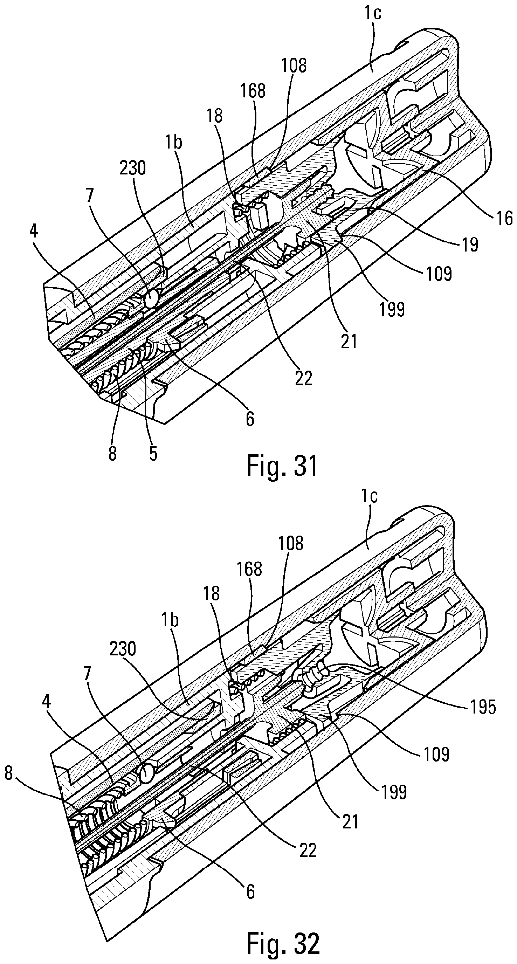

Le ressort 18 s'appuie d'une part sur le corps intermédiaire 1b et d'autre part sur le piston 19, comme visible notamment dans les figures 31 à 34. En variante, ledit ressort pourrait coopérer avec le réservoir.

La clé de verrouillage 20 comporte une tête 21 adaptée à coopérer avec le système retardateur, une tige longitudinale 22, et en embout 23 adapté à coopérer avec la tige de piston 5.

En position avant déclenchement du système retardateur, la tête 21 de la clé de verrouillage 20 est en position de blocage dans laquelle elle coopère avec un évidement axial 190 du piston 19, de sorte que celui-ci est bloqué en translation par ladite clé. Lorsque la tige de piston 5 arrive vers sa position de fin d'injection, elle va coopérer avec l'embout 23 de la clé de verrouillage 20, et ainsi tirer ladite clé axialement vers le bas. De ce fait, la tête 21 de ladite clé de verrouillage 20 sort axialement dudit évidement 190, de sorte que ledit piston 19 n'est plus bloqué en translation par ladite clé 20.

Le ressort 18 sollicite le piston 19 axialement en translation en direction de l'arrière dudit corps supérieur 1c. Tant que le piston 19 est bloqué par ladite clé de verrouillage 20, le système retardateur est donc également bloqué.

Dans l'exemple représenté, avant actionnement du système retardateur, le piston 19 est bloqué contre tout déplacement axial vers le haut par une première paroi tronconique ou inclinée 109 du corps supérieur 1c qui coopère avec des pattes flexibles 199 dudit piston 19. Dans l'exemple représenté, il y a deux pattes flexibles 199 diamétralement opposées, mais on pourrait envisager un nombre différent de premières pattes flexibles, par exemple une seule ou plus de deux pattes. Comme visible notamment sur la figure 31, en position de blocage, ladite tête 21 de la clé de verrouillage 20 empêche la déformation radiale vers l'intérieur desdites pattes flexibles 199 du piston 19. Lorsque ladite tête 21 n'est plus en position de blocage, lesdites pattes flexibles 199 peuvent se déformer radialement vers l'intérieur, sous l'effet du ressort 18 qui pousse ledit piston 19 axialement vers le haut, et du fait de la coopération desdites pattes flexibles 199 avec la première paroi inclinée 109 du corps 1.

Lorsque ladite clé de verrouillage 20 libère ledit piston 19 en fin d'injection, celui-ci est donc déplacé axialement par ledit ressort 18. Le système retardateur est alors déclenché, avec ledit piston 19 se déplaçant axialement dans ledit réservoir 16. Ceci provoque le transfert du fluide hors dudit réservoir 16 à travers un ou plusieurs passage(s) d'écoulement 195 approprié(s). Dans l'exemple représenté, ce passage d'écoulement est formé par un trou central du piston 19, notamment visible sur les figures 31 à 33. Ce trou central prolonge axialement ledit évidement 190 du piston 19. Avantageusement, ce trou central est, avant actionnement du système retardateur, obturé par ladite tête 21 de ladite clé de verrouillage 20, ce qui permet d'assurer l'étanchéité du réservoir 16. La vitesse de déplacement axial du piston 19 subit donc un freinage dû au transfert du fluide hors du réservoir 16 à travers ledit passage d'écoulement 195

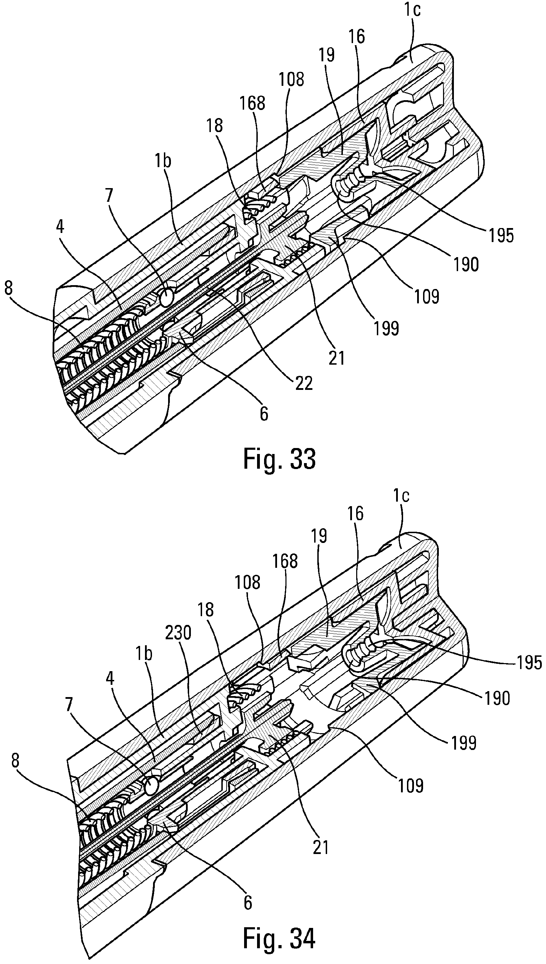

Pendant le déplacement dudit piston 19 dans ledit réservoir 16, ce dernier est bloqué contre tout déplacement axial par des pattes flexibles 168 dudit réservoir 16 qui coopèrent avec une seconde paroi inclinée 108 du corps 1, comme visible sur les figures 31 et 32. Dans l'exemple représenté, il y a deux pattes flexibles 168 diamétralement opposées, mais on pourrait envisager un nombre différent de pattes flexibles, par exemple une seule ou plus de deux pattes. Tant que le piston 19 n'a pas terminé son déplacement axial dans ledit réservoir 16, il empêche la déformation radiale vers l'intérieur desdites pattes flexibles 168 du réservoir 16. Lorsque ledit piston 19 a terminé son déplacement axial dans ledit réservoir 16, il ne bloque plus lesdites pattes flexibles 168, qui peuvent alors se déformer radialement vers l'intérieur, sous l'effet du ressort 18 qui pousse l'unité formée par ledit piston 19 et ledit réservoir 16 axialement vers le haut, et du fait de la coopération desdites pattes flexibles 168 avec la seconde paroi inclinée 108 du corps 1. Ceci est notamment visible sur la figure 34.

Lorsque ledit piston 19 a terminé son déplacement axial dans ledit réservoir 16, celui-ci est donc libéré de son blocage, et l'unité formée du piston 19 et du réservoir 16 est alors projetée par le ressort 18 axialement contre le fond du corps supérieur 1c, comme illustré sur la figure 34. La partie d'indication 160 du réservoir 16 est alors disposée face à la fenêtre d'indication 11 du corps 1. Ce déplacement non freiné ou projection de ladite unité permet aussi de générer une indication sonore, par contact entre le réservoir 16 et/ou le piston 19 avec le corps 1. Cette indication sonore peut être réalisée au niveau de ladite partie d'indication 160 ou sur une autre partie de l'unité formée par le piston 19 et le réservoir 16, adaptée à coopérer avec une partie appropriée du corps 1. Il est à noter que le choc générant l'indication sonore peut aussi fournir une indication tactile en générant une vibration de l'autoinjecteur dans la main de l'utilisateur, utile notamment pour les malentendants.

Dans les deux modes de réalisation décrits ci-dessus, le fluide utilisé dans le système retardateur peut être de tout type approprié, par exemple une graisse. L'utilisation d'un fluide ayant des propriétés viscoélastiques est avantageuse.

Le dispositif retardateur permet donc de décaler d'un temps prédéterminé le moment où l'indicateur va indiquer la fin d'utilisation, à partir du moment où la phase d'injection est terminée.

Une phase complète d'actionnement de l'autoinjecteur va être décrite ci-après.

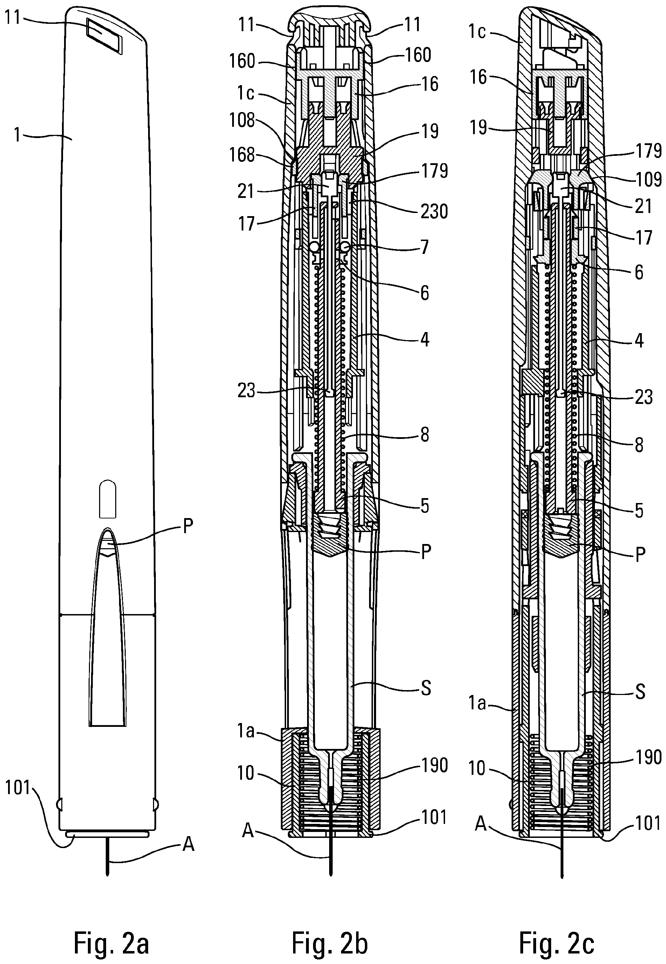

Lorsque l'utilisateur veut utiliser l'autoinjecteur, il prend le dispositif, par exemple au niveau du corps 1 et il appuie le manchon actionneur 10, qui au repos, dans sa première position projetée, fait saillie hors du corps inférieur 1, contre la partie du corps où il veut réaliser l'injection. Sur les figures 1a, 1b et 2a, 2b d'une part et 20a, 20b et 21a, 21b d'autre part, on voit que la pression exercée par l'utilisateur sur le manchon actionneur 10 provoque le coulissement de celui-ci vers l'intérieur du corps 1, ce qui découvre l'aiguille et donc son piquage en raison de la pression exercée par l'utilisateur sur l'autoinjecteur.

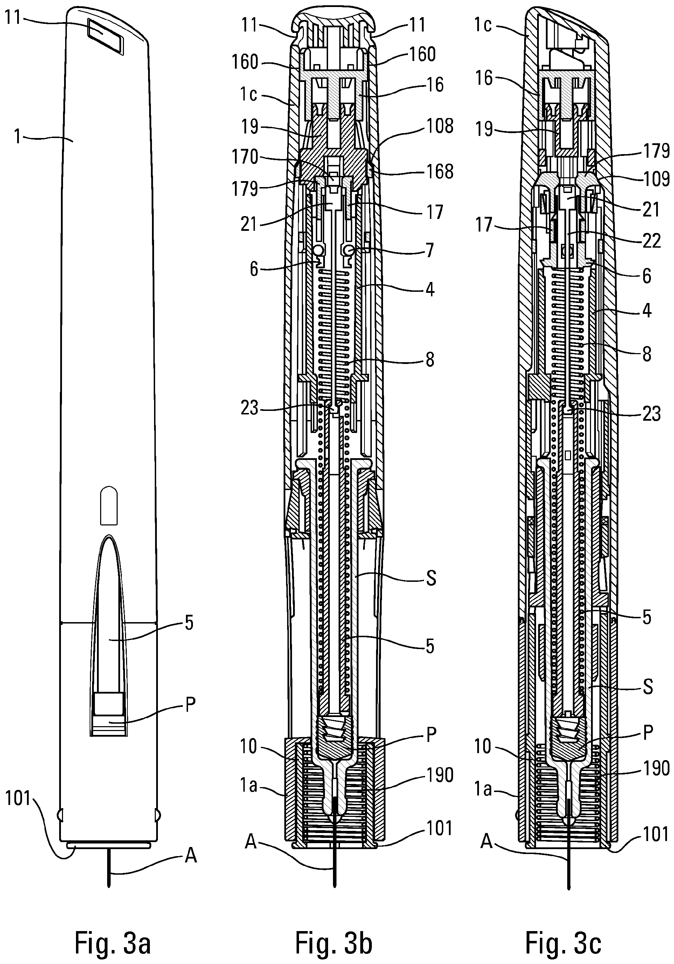

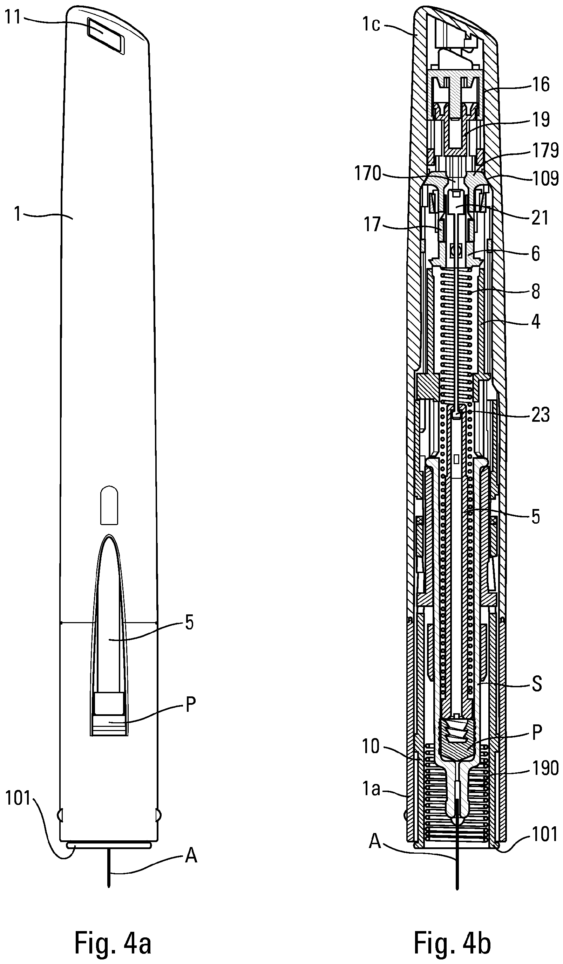

Lorsque le manchon actionneur 10 atteint sa position d'actionnement, qui est sa position d'extrémité à l'intérieur du corps 1, il provoque le déclenchement de la phase d'injection, ce qui est représenté sur les figures 3a, 3b et 4a, 4b d'une part et 22a, 22b et 23a, 23b d'autre part. On constate que la tige de piston 5 coulisse à l'intérieur de la seringue A en poussant le piston P de celle-ci sous l'effet du ressort d'injection 8. Le produit est donc distribué.

A la fin de l'injection, le système retardateur est déclenché, de sorte que le dispositif d'indication ne sera actionné qu'après un temps de retard prédéterminé.

Après indication de la fin d'utilisation, lorsque l'utilisateur retire l'autoinjecteur du site d'injection, le manchon actionneur 10 est à nouveau déplacé hors du corps 1 vers la position de fin d'utilisation, qui est sa seconde position projetée, sous l'effet du ressort du manchon actionneur, avec un verrouillage dudit manchon actionneur 10, ce qui garantit une sécurité absolue pour l'utilisateur et évite tout risque de blessures avec l'aiguille après utilisation du dispositif.

Il est à noter que les première et seconde positions projetées du manchon actionneur, qui, dans l'exemple représenté, sont des positions différentes, pourraient éventuellement être identiques.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à un mode de réalisation avantageux, il est entendu que celui-ci n'est pas limitatif. En particulier, le manchon actionneur et/ou la serrure d'injection et/ou le dispositif retardateur et/ou le dispositif d'indication sonore et/ou tactile pourrai(en)t être réalisés différemment. Le piquage de l'aiguille et/ou une rétractation de l'aiguille après injection pourrait être commandé par un ou plusieurs bouton(s). D'autres modifications sont également possibles pour l'homme du métier sans sortir du cadre de la présente invention tel que défini par les revendications annexées.

## Revendications

1. Autoinjecteur comportant:
- un corps (1) recevant un réservoir (S), ledit réservoir (S) contenant du produit fluide et comportant un piston (P), tel qu'une seringue pré-remplie,
- une tige de piston (5) adaptée à coopérer avec le piston (P) dudit réservoir (S), ladite tige de piston (5) étant déplaçable par un ressort d'injection (8) entre une position chargée et une position d'injection dans laquelle ladite tige de piston (5) a déplacé le piston (P) du réservoir (S) pour injecter le produit fluide dans un site d'injection,
- un dispositif d'indication visuel, sonore et/ou tactile pour indiquer à l'utilisateur qu'il peut retirer ledit autoinjecteur dudit site d'injection,
ledit autoinjecteur comportant un système retardateur pour retarder l'actionnement dudit dispositif d'indication visuel, sonore et/ou tactile par rapport à la fin de l'injection, ledit système retardateur comportant un réservoir (16) contenant un fluide, un piston (19) étant disposé dans ledit réservoir (16), l'un parmi ledit réservoir (16) et ledit piston (19) étant, pendant l'actionnement dudit système retardateur, déplaçable en translation dans ledit corps (1) et l'autre parmi ledit réservoir (16) et ledit piston (19) étant, pendant l'actionnement dudit système retardateur, fixe par rapport audit corps (1), le déplacement en translation de l'un par rapport à l'autre déplaçant ledit fluide hors dudit réservoir (16), à travers au moins un passage d'écoulement (161; 195), de telle sorte que ledit déplacement en translation est freiné, **caractérisé en ce que** ledit système retardateur comprend ledit réservoir (16) contenant ledit fluide, ledit piston (19), un ressort retardateur (18) différent dudit ressort d'injection (8) fixé d'une part audit piston (19) et d'autre part audit corps (1), une clé de verrouillage (20) et ladite tige de piston (5), ladite clé de verrouillage (20) comportant une tête (21), une tige longitudinale (22) et en embout (23) adapté à coopérer avec la tige de piston (5), ladite tête (21) de la clé de verrouillage (20), avant déclenchement du système retardateur, étant en position de blocage dans laquelle elle coopère avec un évidement (190) dudit piston (19).

2. Autoinjecteur selon la revendication 1, dans lequel, pendant l'actionnement dudit système retardateur, ledit piston (19) est déplaçable en translation dans ledit corps (1) et ledit réservoir (16) est fixe.

3. Autoinjecteur selon la revendication 2, dans lequel, pendant l'actionnement dudit système retardateur, ledit réservoir (16) est bloqué en translation par une seconde partie de paroi tronconique ou inclinée (108) dudit corps (1), coopérant avec au moins une patte flexible (168) dudit réservoir (16), ledit piston (19), pendant l'actionnement dudit système retardateur, empêchant la déformation radiale vers l'intérieur desdites pattes flexibles (168).

4. Autoinjecteur selon la revendication 3, dans lequel, après actionnement dudit système retardateur, ledit piston (19) s'est déplacé axialement par rapport à ladite au moins une patte flexible (168) dudit réservoir (16) et ne bloque plus sa déformation radiale vers l'intérieur, de sorte que ledit réservoir (16) n'est plus bloqué en translation dans ledit corps (1).

5. Autoinjecteur selon l'une quelconque des revendications précédentes, dans lequel ledit au moins un passage d'écoulement (161) est formé par une gorge axiale du réservoir (16).

6. Autoinjecteur selon l'une quelconque des revendications 1 à 4, dans lequel ledit au moins un passage d'écoulement (195) est formé par un trou central du piston (19).

7. Autoinjecteur selon l'une quelconque des revendications précédentes, dans lequel, lorsque la tige de piston (5) arrive vers sa position de fin d'injection, elle coopère avec l'embout (23) de la clé de verrouillage (20) pour tirer ladite clé de verrouillage (20) axialement vers le bas hors de sa position de blocage, de sorte que ledit piston (19) n'est alors plus bloqué en translation par ladite clé de verrouillage (20).

8. Autoinjecteur selon l'une quelconque des revendications précédentes, dans lequel ledit piston (19) comporte des pattes flexibles (199) coopérant, avant actionnement du système retardateur, avec une première paroi tronconique ou inclinée (109) du corps (1), la tête (21) de la clé de verrouillage (20), en position de blocage, empêchant la déformation radiale vers l'intérieur desdites pattes flexibles (179).

9. Autoinjecteur selon l'une quelconque des revendications précédentes, dans lequel ledit autoinjecteur comporte un manchon actionneur (10) comportant une extrémité de contact (101) destinée à venir en contact avec le corps de l'utilisateur, ledit manchon actionneur (10) s'étendant au moins partiellement à l'intérieur dudit corps (1) et étant déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur.

10. Autoinjecteur selon l'une quelconque des revendications précédentes, dans lequel ledit réservoir (S) comporte une aiguille (A) à travers laquelle ledit produit fluide est injecté dans ledit site d'injection.

## Patentansprüche

1. Autoinjektor, folgendes umfassend:
Einen Körper (1), der einen Vorratsbehälter (S) in sich aufnimmt, wobei der Vorratsbehälter (S) ein Fluidprodukt enthält und einen Kolben (P), beispielsweise eine vorgefüllte Spritze, umfasst,
eine Kolbenstange (5), die geeignet ist, mit dem Kolben (P) des Vorratsbehälters (S) zusammenzuwirken, wobei die Kolbenstange (5) durch eine Injektionsfeder (8) zwischen einer geladenen Stellung und einer Injektionsstellung hin und her bewegbar ist, wobei bei letzterer die Kolbenstange (5) den Kolben (P) des Vorratsbehälters (S) zur Injektion des Fluidprodukts in eine Injektionsstelle bewegt hat,
eine optische, akustische und/oder taktile Anzeigevorrichtung, mit der dem Benutzer angezeigt wird, dass der Autoinjektor von der Injektionsstelle entfernt werden kann,
wobei der Autoinjektor ein Verzögerungssystem zum Verzögern der Betätigung der optischen, akustischen und/oder taktilen Anzeigevorrichtung bezüglich des Endes der Injektion umfasst,
wobei das Verzögerungssystem einen Vorratsbehälter (16) mit einem darin enthaltenen Fluid umfasst, ein Kolben (19) in dem Vorratsbehälter (16) angeordnet ist, entweder der Vorratsbehälter (16) oder der Kolben (19) während der Betätigung des Verzögerungssystems in dem Körper (1) translatorisch bewegbar ist und die jeweils andere Komponente, d.h. der Vorratsbehälter (16) bzw. der Kolben (19), während der Betätigung des Verzögerungssystems in Bezug auf den Körper (1) ortsfest ist, wobei die translatorische Bewegung der einen Komponente in Bezug auf die andere das Fluid aus dem Vorratsbehälter (16) durch mindestens einen Durchflusskanal (161; 195) hindurch verdrängt, sodass die translatorische Bewegung abgebremst wird, **dadurch gekennzeichnet, dass** das Verzögerungssystem den das Fluid enthaltenden Vorratsbehälter (16), den Kolben (19), eine gegenüber der Injektionsfeder (8) unterschiedliche Verzögerungsfeder (18), die einerseits an dem Kolben (19) und andererseits an dem Körper (1) befestigt ist, einen Verriegelungsschlüssel (20) und die Kolbenstange (5) umfasst, wobei der Verriegelungsschlüssel (20) einen Kopf (21), eine Längsstange (22) und ein Endstück (23) aufweist, das geeignet ist, mit der Kolbenstange (5) zusammenzuwirken, wobei sich der Kopf (21) des Verriegelungsschlüssels (20) vordem Auslösen des Verzögerungssystems in einer Verriegelungsstellung befindet, in der er mit einer Ausnehmung (190) des Kolbens (19) zusammenwirkt.

2. Autoinjektor nach Anspruch 1, bei dem während der Betätigung des Verzögerungssystems der Kolben (19) in dem Körper (1) translatorisch bewegbar ist und der Vorratsbehälter (16) ortsfest ist.

3. Autoinjektor nach Anspruch 2, bei dem während der Betätigung des Verzögerungssystems der Vorratsbehälter (16) durch einen zweiten kegelstumpfförmigen oder schrägen Wandabschnitt (108) des Körpers (1) in seiner Verschiebung blockiert wird, in Zusammenwirkung mit mindestens einem flexiblen Vorsprung (168) des Vorratsbehälters (16), wobei der Kolben (19) während der Betätigung des Verzögerungssystems eine radiale Verformung der flexiblen Vorsprünge (168) nach innen verhindert.

4. Autoinjektor nach Anspruch 3, bei dem sich nach Betätigung des Verzögerungssystems der Kolben (19) axial in Bezug auf den mindestens einen flexiblen Vorsprung (168) des Vorratsbehälters (16) bewegt hat und dessen radiale Verformung nach innen nicht mehr verhindert, sodass der Vorratsbehälter (16) nicht mehr bezüglich seiner translatorischen Bewegung im Körper (1) blockiert ist.

5. Autoinjektor nach einem der vorhergehenden Ansprüche, bei dem der mindestens eine Durchflusskanal (161) durch eine axiale Nut des Vorratsbehälters (16) gebildet wird.

6. Autoinjektor nach einem der vorhergehenden Ansprüche 1 bis 4, bei dem der mindestens eine Durchflusskanal (161) durch ein zentrales Loch im Kolben (19) gebildet wird.

7. Autoinjektor nach einem der vorhergehenden Ansprüche, bei dem, wenn die Kolbenstange (5) ihre Injektionsendstellung erreicht, diese mit dem Endstück (23) des Verriegelungsschlüssels (20) zusammenwirkt, um den Verriegelungsschlüssel (20) axial nach unten aus dessen Verriegelungsstellung herauszuziehen, sodass der Kolben (19) nicht mehr durch den Verriegelungsschlüssel (20) bezüglich seiner translatorischen Bewegung blockiert ist.

8. Autoinjektor nach einem der vorhergehenden Ansprüche, bei dem der Kolben (19) flexible Vorsprünge (199) aufweist, die vor der Betätigung des Verzögerungssystems mit einer ersten kegelstumpfförmigen oder schrägen Wand (109) des Körpers (1) zusammenwirken, wobei der Kopf (21) des Verriegelungsschlüssels (20) in der Verriegelungsstellung eine radiale Verformung der flexiblen Vorsprünge (179) nach innen verhindert.

9. Autoinjektor nach einem der vorhergehenden Ansprüche, bei dem der Autoinjektor eine Betätigungshülse (10) mit einem Kontaktende (101) umfasst, das mit dem Körper des Benutzers in Kontakt gebracht wird, wobei sich die Betätigungshülse (10) zumindest teilweise innerhalb des Körpers (1) erstreckt und relativ zu dem Körper (1) zwischen ausgefahrenen Stellungen, in denen die Betätigungshülse (10) zumindest teilweise aus dem Körper (1) herausragt, und einer Betätigungsstellung, in der die Betätigungshülse (10) axial in Richtung des Inneren des Körpers (1) verschoben ist, bewegbar ist, wobei sich die Betätigungshülse (10) vor der Betätigung des Autoinjektors in einer ersten ausgefahrenen Stellung und nach der Betätigung des Autoinjektors in einer zweiten ausgefahrenen Stellung befindet.

10. Autoinjektor nach einem der vorhergehenden Ansprüche, bei dem der Vorratsbehälter (S) eine Nadel (A) umfasst, mit der das Fluidprodukt in die Injektionsstelle injiziert wird.

## Claims

1. An autoinjector comprising:
• a body (1) receiving a reservoir (S), said reservoir (S) containing fluid and including a piston (P), such as a pre-filled syringe,
• a piston rod (5) that is adapted to co-operate with the piston (P) of said reservoir (S), said piston rod (5) being movable by an injection spring (8) between a primed position and an injection position in which said piston rod (5) has moved the piston (P) of the reservoir (S) so as to inject the fluid into an injection site,
• a visual, audible, and/or tactile indicator device for indicating to the user that said autoinjector may be removed from said injection site,
said autoinjector including a retarding system for delaying the actuation of said visual, audible and/or tactile indicator device relative to the end of injection, said retarding system including a dashpot (16) containing a fluid, a piston (19) being arranged in said dashpot (16), one of said dashpot (16) and of said piston (19) being, movable in translation in said body (1) during actuation of said retarding system, and the other of said dashpot (16) and of said piston (19) being, stationary relative to said body (1), during actuation of said retarding system, the movement in translation of one relative to the other moving said fluid out from said dashpot (16), through at least one flow passage (161; 195), such that said movement in translation is braked, **characterized in that** said retarding system includes said dashpot (16) containing said fluid, said piston (19), a retarding spring (18) that is different from said injection spring (8) and that is fastened firstly to said piston (19) and secondly to said body (1), a locking key (20) and said piston rod (5), said locking key (20) comprising a head (21), a longitudinal rod (22) and an endpiece (23) that is adapted to co-operate with the piston rod (5), said head (21) of the locking key (20) being in its blocking position before triggering the retarding system, in which position it co-operates with a recess (190) of said piston (19).

2. An autoinjector according to claim 1, wherein, during actuation of said retarding system, said piston (19) is movable in translation in said body (1), and said dashpot (16) is stationary.

3. An autoinjector according to claim 2, wherein, during actuation of said retarding system, said dashpot (16) is prevented from moving in translation by a second frustoconical or sloping wall portion (108) of said body (1), that co-operates with at least one flexible tab (168) of said dashpot (16), said piston (19), during actuation of said retarding system, preventing said flexible tabs (168) from deforming radially inwards.

4. An autoinjector according to claim 3, wherein, after actuating said retarding system, said piston (19) is moved axially relative to said at least one flexible tab (168) of said dashpot (16) and no longer prevents it from deforming radially inwards, such that said dashpot (16) is no longer prevented from moving in translation in said body (1).

5. An autoinjector according to any preceding claim, wherein said at least one flow passage (161) is formed by an axial groove in the dashpot (16).

6. An autoinjector according to any one of claims 1 to 4, wherein said at least one flow passage (195) is formed by a central hole in the piston (19).

7. An autoinjector according to any preceding claim, wherein, when the piston rod (5) arrives towards its end-of-injection position, it co-operates with the endpiece (23) of the locking key (20) so as to pull said locking key (20) axially downwards out from its blocking position, such that said piston (19) is thus no longer prevented from moving in translation by said locking key (20).

8. An autoinjector according to any preceding claim, wherein said piston (19) includes flexible tabs (199) that before actuation of the retarding system, co-operate with a first frustoconical or sloping wall (109) of the body (1), the head (21) of the locking key (20), in its blocking position, preventing said flexible tabs (179) from deforming radially inwards.

9. An autoinjector according to any preceding claim, wherein said autoinjector includes an actuator sleeve (10) that includes a contact end (101) for coming into contact with the user's body, said actuator sleeve (10) extending inside said body (1) at least in part and being movable relative to said body (1) between projecting positions, in which said actuator sleeve (10) projects out from said body (1) at least in part, and an actuated position in which said actuator sleeve (10) is moved axially into said body (1), said actuator sleeve (10) being in a first projecting position before actuation of the autoinjector and in a second projecting position after actuation of the autoinjector.

10. An autoinjector according to any preceding claim, wherein said reservoir (S) includes a needle (A) through which said fluid is injected into said injection site.
